# EUROPEAN PATENT APPLICATION

(11) **EP 1 357 130 A1**
(43) Date of publication of application: **29.10.2003**
(21) Application number: 01997505.1
(22) Date of filing: 23.11.2001
(51) Int. Cl.: C07K 14/50, A61K 38/18, A61P 17/02

(54) **BASIC FIBROBLASTIC GROWTH FACTOR WITH A SPECIFIC COLLAGEN BINDING DOMAIN**

(30) Priority: 23.11.2000 ES 200002811
(71) Applicant: Universidad de Malaga, 29071 Malaga (ES)
(72) Inventor: ANDRADES GOMEZ, José Antonio, E-29071 Malaga (ES); BECERRA RATIA, José, E-29071 Malaga (ES); HALL, Frederick, L., E-29071 Malaga (ES); NIMNI, Marcel, E., E-29071 Malaga (ES)
(74) Representative: Maldonado Jordan, Julia
(86) International application number: ES0100449
(87) International publication number: WO02042331

(57) **Abstract**

The invention relates to a basic fibroblastic growth factor (bFGF) with a specific collagen binding domain that is obtained by recombinant DNA technology, which includes a 10 amino acid fragment corresponding to the modified collagen binding factor of the von Willebrand factor (vWF). The new controlled release factor is used as a therapeutic agent in the cicatrization of wounds and in other tissue repair processes.

## Description

### SCOPE OF THE ART

The present invention is applicable within the biomedical industry dedicated to the manufacturing of agents that intervene in the repair of tissue in general and more specifically, in the cicatrizing processes of wounds.

### STATE OF THE ART

The fibroblastic growth factors (FGFs) were isolated for the first time in the 1970's as from bovine brain extracts, based on their mitogenic and angiogenic character. Subsequent studies established that FGFs constitute a family of twenty structurally related monomeric proteins (molecular weight between 16000 and 18000), with diverse activities, and are produced at some moment during the development of tissues such as the epithelium, muscles, connective and nervous tissue. Due to the fact that FGFs have lost the signal peptide sequence, which determines in other growth factors its extracellular secretion via the rugose endoplasmic reticulum / Golgi apparatus, it has been suggested that its liberation outside the cell could occur following an independent exocytic pathway (see Mignatti P. and collaborators, J. Cell Biochem. (1991), 47: 201-207). Injuries due to wounds cause an important FGF liberation, since the plasmatic membrane of the damaged cells becomes permeable to large molecules, allowing a free diffusion towards the extracellular space (see Mutsukrishnan L. and collaborators, J. Cell Physiol. (1991), 148: 1-16).

FGFs belong to the group of growth factors with a heparin binding affinity, and it is due to this that they are joined to sulphate heparin proteoglucans of the basal membrane, where they are stored. From such bindings, FGFs are liberated by means of the heparinase action (see Saskela O. and collaborators, J. Cell Biol. (1990), 110: 767-775).

Immunocytochemical techniques in rat aorta reveal that a FGF, the basic FGF (bFGF or FGF-2), is stored in the cytoplasm of endothelial cells and of smooth muscle (see Villaschi S. and collaborators, Am. J. Pathol. (1993), 143: 181-190). Studies carried out on rat aorta wounds show that a liberation of bFGF is produced from its storage locations. In fact, the bFGF levels in vascular endothelial cells in culture, are the highest during the first days, gradually decreasing until they become undetectable when the micro vessels stop growing. The treatment of the cultures with anti-bFGF antibodies causes 40% of the angiogenic response inhibition.

In addition to the first effects observed on the cellular and angiogenesis response, FGFs regulate the survival, apoptosis, adhesion, motility and cellular differentiation. These effects of the FGFs on the cellular functions depend on the biochemical condition and on the environment of their target cells. It has been demonstrated that the bFGF carries out an important role in the first stages of the tissue repair processes (see Pierce G.F. and collaborators, Am. J. Pathol. (1992), 140: 1375-1388) and is a good mitogen for condrocites and osteoblasts, stimulating the cellular proliferation inside the fracture callus, as well -as the growth and development of bone tissue inside the synthetic hydroxyapatite (see Wang J-S. and collaborators, J. Orthop. Res. (1996), 14: 316-323; and Clin. Ortohop. Rel. Res. (1996), 333; 252-260).

The biology of human bFGF is complex. Various isoforms of the factor (of 18, 22, 23 and 24 kDa) may be expressed as from a simple ARNm transcriptor (see Florkiewicz R.Z. and collaborators, Proc. Natl. Acad. Sci. USA (1989), 86; 3978-3981). These bFGF isoforms appear in different locations of the core (the 22, 23 and 24 kDa), of the cytoplasm or of the cellular surface (the 18 kDa). The three dimensional structure of the human bFGF determined by X-ray crystallography, suggests a globular formation that shows separated binding domains to its receptor and to the heparin (see Nimni M.E., Biomaterials (1997), 18; 1201-1225).

In spite of their great angiogenic and mitogenic power, the bFGF rapidly degrades when injected or ingested, losing in a short time up to a 99% of its mitogenic activity. The preservation and stabilization of the bFGF is obtained when the factor is bound to heparin-sepharose spheres. This allows its prolonged storage, repeated handling, as well as its slow and controlled liberation (see Edelman E.R. and collaborators. Biomaterials (1991), 12; 619-626).

The family of FGFs is a large cytoquinine group that exerts a profound influence in the physiology of the cicatrization of wounds. Its manner of action on the cicatrization of wounds includes the modulation of mother cells populations, as well as the expression of specific genes that codify for the matrix proteins, cellular receptors, matrix proteinase and protease inhibitors.

Numerous studies on animals have demonstrated the efficiency of the exogenous FGF in aiding the cicatrization of wounds, which has also lead to their clinical use, to be employed on wounds produced as a consequence of surgery, for bone repair, as well as for the treatment of ulcers (foot, gastric) and burns on diabetic patients. The systematic application of a dose of bFGF at the moment the wound is caused, has demonstrated the capability of accelerating the repair of the tissue, suggesting that one single dose may increase the recovery rate of the wound.

Clinical studies performed to date, using bFGF as therapeutical agent, have not gone very far due to the limited availability of this factor in the amounts required. Availability of large amounts of pharmaceutical grade bFGF are required, free from easily transmitted contaminants, omnipresent on products extracted from animals and in particular, from human sources. Consequently, it is very desirable to develop mechanisms for the preparation of large amounts of synthetic, mature bFGF where, in addition, any possibility of dangerous contaminating materials being present in the final product is eliminated. Finally, the biologically active protein shall be designed so that it specifically reaches locations of the wound where its cicatrization is intended.

### DESCRIPTION OF THE INVENTION

The genetic design and expression on a large scale of a human recombinant bFGF in the *Escherichia coli* bacteria has been carried out in the present invention, as well as its purification and re-naturalization, until it reaches its maximum biological activity. The advantage presented by said factor is that an auxiliary modified amino acid domain has been incorporated, of high specific collagen binding affinity, that confers unique and exclusive properties. In this manner, this growth factor, called rhbFGF-F2, may be stored during prolonged periods of time and directed towards specific locations where its actuation is required (for example repair/cicatrization of wounds), controlling its liberation and preserving its biostability, thus accentuating its potential as possible therapeutic agent due to its role in the cicatrization of wounds and on other tissue repair processes.

The present invention is useful for generating large amounts of biologically active hbFGF as from prokaryote microorganisms. This system, contrary to the development in eukaryote cells as from ARNm, where the availability of material is the restricting factor, offers a practically inexhaustible source for the expression, synthesis and secretion of proteins, which only depends on the cellular volume (bacterial) with which the culture is commenced. Throughout the bFGF manufacturing technology period itself, a method has been developed that especially increases the performance as regards the amount obtained of purified native proteins, the accuracy during the proteic renaturation process, the efficiency and the shortening of the dialysis procedure, and the achievement of the maximum biological activity rate.

Likewise, the present invention is useful for manufacturing hbFGF under the previously described conditions and for incorporating in it an auxiliary amino acidic molecular domain in its primary structure (corresponding to the modified domain of the von Willebrand factor, of specific binding to type I collagen). The aim of this modification is to make the resultant fusion protein rhbFGF-F2 reach specific cellular-tissular targets, so that, there, it can perform the desired function.

Bearing in mind the known role that the FGF performs in the first stages of the tissular repair process (see Pierce G.F. and collaborators, Am. J. Pathol. (1992), 140: 1375-1388), as has been indicated in bone repair (see Wang J-S. and collaborators, J. Orthop. Res. (1996), 14: 316-323; and Clin. Ortop. Rel. Res. (1996), 333: 252-260), and with special attention to the cicatrization rate of the wounds, the present invention offers an rhbFGF-F2 that significantly accelerates the cicatrization of skin wounds with total or partial incisions. In this respect, the use of this patent is reinforced even more, when the produced FGF is ectopically applied, mixed with a type I collagen gel, causing a quicker wound cicatrization rate.

On the other hand, it has been described, that the bFGF regulates the synthesis and deposit of various components of the extracellular matrix (MEC) (see, for example, patent application ES 2 132 082 T3), and it is known, that in diabetic individuals, the wound cicatrization process is repressed, first in its inflammatory reaction stage, and secondly in a subsequent remodelation stage of the MEC (see Gospodarowicz D. and collaborators, Endocr. Rev. (1987), 8: 95-114). In this sense, the present invention presents a clinical utility as regards the fact that an rhbFGF-F2 is developed which, combined with a type I collagen gel and applied ectopically on total or partial wounds, reduces the cicatrization time on diabetic animals until said time is brought to terms that are comparable to normal animals.

### BRIEF DESCRIPTION OF THE DRAWINGS

Figure 1 is the representation of (A) cloning vector TA and expression vector pET28b; (B) the fusion proteins: rhbFGF-F2, that incorporates the WREPSFMALS amino acidic sequence of the von Willebrand factor (vWF), and the rhbFGF-F1 that lacks said sequence.
Figure 2 is the SDS gel of expression and purification of the fusion proteins. Path 1 indicates the molecular weight of the marker. Paths 2 and 5 show proteins rhbFGF-F1 and -F2 respectively, before the induction. Paths 3 and 6 correspond to proteins rhbFGF-F1 and -F2 respectively, after the induction. Paths 4 and 7 show the proteins in pure condition. The slight offset of the strip in path 7 as regards the strip of path 4, corresponds to the greater molecular weight of rhbFGF-F2-introduced by the amino acidic sequence corresponding to the von Willebrand factor.
Figure 3 shows (A) a SDS gel indicating the different oxide-reduction conditions tested during the protein renaturation, and the one that offers the greatest performance as regards the protein obtention, and (B) shows how said redox condition is the one that offers the greatest biological activity to each protein.
Figure 4 represents the biological activity, in terms of cellular proliferation, of the rhbFGE-F2 under the different dialysis conditions tested. Buffer A with 250 mM of NaC1 and 20% of saccharose favours the maximum biological activity of the protein.
Figure 5 shows how the biological activity, in terms of cellular proliferation, of the rhbFGF-F1 (open circle) and of the rhbFGF-F2 (closed circle), obtained under optimum dialysis conditions, is comparable to the biological activity of the commercial protein (triangle).
Figure 6 represents the binding power of the fusion proteins to the type I collagen. Whilst ³H-collagen bound to rhbFGF-F1 is totally and rapidly eluated, the ³H-collagen bound to rhbFGF-F2 requires a strong urea gradient in order to be eluated.
Figure 7 shows (A) a ³H-rhbFGF-F1 and ³H-rhbFGF-F2 dose-dependent kinetic binding to type I collagen, after the digestion of collagen with 0.5 ml of collagenase (100 U/ml). The collagen binding affinity demonstrated by the rhbFGF-F1 obeys to non specific interactions. In (B) it is observed that both fusion proteins continue keeping their biological activity, in terms of cellular proliferation, after being liberated from type I collagen.
Figure 8 is a bar chart corresponding to the macroscopic examination results of the wounds cicatrization rate, both in normal and in diabetic-affected rats. The ectopic treatment of the wounds is indicated in each case.

### DETAILED DESCRIPTION OF THE INVENTION

The procedure followed in the present invention for the preparation of the basic fibroblastic growth factor, with a specific collagen binding domain, that is the object of the present invention and the evaluation of its applicability, basically consists of the following stages:
- *bFGF genetic construction.*
- *FGFs proteic expression.*
- *FGFs solubilisation and purification*
- *FGFs renaturation*
- *Biological activity of the soluble and collagen-bound proteic forms.*
- *Analysis of the specific collagen binding of rhbFGF-F2.*
- *Applicability for the cicatrization of wounds.*

As a preferred embodiment of the invention, the methods and procedures followed in these basic stages as well as the results obtained and the most relevant conclusions derived from the same are herewith described in detail.

### Methods and procedures

### Genetic construction of the bFGF

Cytoplasmic ARN (ARNc) of human umbilical endothelial cells was obtained, and was transcribed to a simple cytoplasmic DNA chain (DNAc) using anti-direction primers corresponding to the region that codes for the bFGF. A PCR amplification was performed on the DNAc and the resultant products were separated by electrophoresis in agarose. The strips obtained were purified from the agarose gel by means of the Geneclean kit (Bio 101) and introduced in a vector following the TA cloning strategy (Invitrogen). The clones selected by the colour were isolated and analysed by means of restriction mapping, followed by a determination of the nucleotide sequence. Specifically, the primer used for the construction of the bFGF, the fusion protein to which the high collagen affinity binding domain is incorporated, was one of 50 nucleotides with sequence 5' -CAT ATG TGG CGC GAA CCG AGC TTC ATG GCT CTG AGC GGT GCT AGC ATG GCA G-3', designed for inclusion of two parts: 7 nucleotides corresponding to the bFGF sequence bound to 30 nucleotides that code for a modified peptide with collagen binding capacity (see Tuan T.L. and collaborators, Connect. Tissue Res. (1996), 34: 1-9). The resultant construction is introduced in the expression vector pET28b (Novagen), which possesses a purification histidinic sequence in its amino terminal.

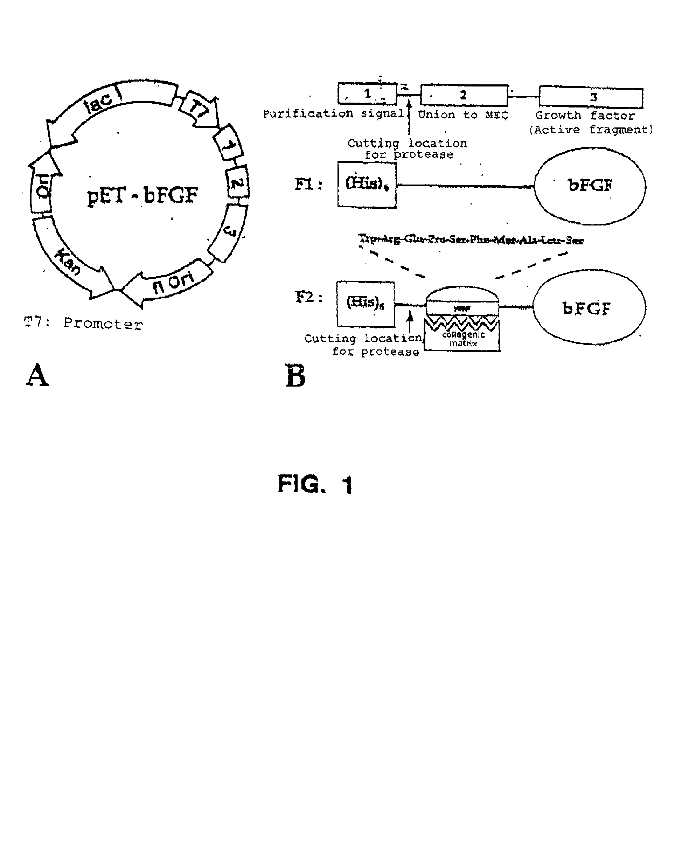

In addition to the construction that incorporates the specific collagen binding domain, pET-hbFGF-F2, we have also developed the one that does not include said domain ("standard" sequence), pET-hbFGF-F1, in order that it may serve at all moments as control of the protocol of design, expression, purification and fusion factor biological activity check. Both F1 and F2 constructions, were maintained in an *Escherichia coli* stock marked in blue XL (Figure 1). The orientation and reading of the sequence of each insert was confirmed by means of manual DNA nucleotidic sequentiation analysis, by the modified dideoxide chain termination method (USB)

### FGFs proteic expression

For the recombination proteins expression, the pET-hbFGF-F1 and pET-hbFGF-F2 constructions were transformed in chain BL21(DE3) of *E. coli.* A simple clone was inoculated in a primary culture that was diluted in order to obtain a final volume of 500 ml with half 2YT, which contained kanamycin (100 µg/ml). After 2-3 hours growth of the culture and after reaching an appropriate growth level, (optical density A600 from 0.7 to 1.0), the proteins were induced in the presence of 1 mM of IPTG during 5 hours at 37°C. The bacterial decanters were obtained by centrifugation at 5000 g during 10 minutes, washed with buffer A (20 mM Tris-HCl, 250 mM NaCl, 0.05% NP-40, pH 8.0) and smoothed with 0.4 mg/ml of lysozyme, 5 ng/ml of DNAasa, 0.8 mM PMSF and 10 mM of ß-mercaptoethanol in 1/10 of the original cellular suspension volume. The lysis was aided by sonication and homogenisation with a Polytron Brinkmann at 25K during 2 cycles of 30 seconds each, followed by centrifugation at 10000 g during 20 minutes at 4°C, to obtain the bacterial inclusion bodies by decantation.

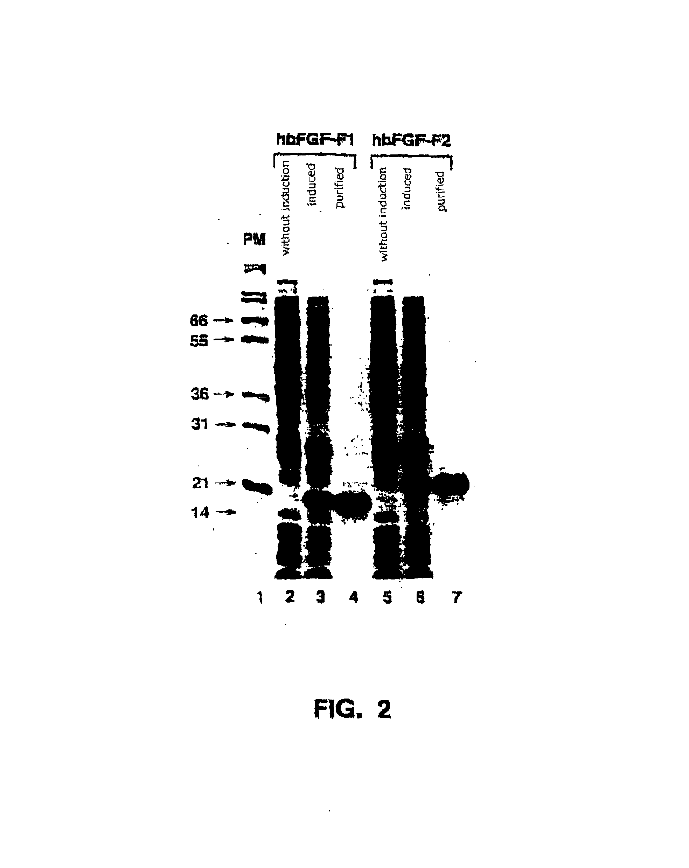

It was determined by electrophoresis in SDS-PAGE gel (Figure 2) that, in the presence of 1 mM of IPTG, the rhbFGFs expression levels reached 50% of the total cellular protein.

### Solubilisation and purification of the FGFs

The inclusion bodies were solubilised in 6 M of guanidine-HC1, 0.1 M phosphate buffer, pH 8.0, at room temperature in 1/10 of the original cells volume, leaving to decant at room temperature during 1.5 hours with sporadic agitations. The solubilised fusion proteins were separated from the insoluble impurities by centrifugation at 20000g.

The purification of the solubilised fusion proteins was performed by Ni-NTA chromatography (Qiagen) : after loading the column with the solubilisation and leaving to rest the sepharose pellets during 30 minutes, washing of the column was performed with 2 volumes of urea 8 M in buffer A, pH 8.0 followed by 2 volumes of urea 8M in buffer A, pH 6.5. The recombinant proteins (F1 and F2) were eluated with 8 M of urea in buffer A, pH 4.0. The proteic concentration was determined by the Bradford method (BioRad) using bovine albumin as reference standard.

### Renaturation of the FGFs

After purification of the proteins by affinity chromatography, the samples were diluted in order to obtain a concentration of 0.20 mg/ml in 8 M of urea, pH 8.0. The oxide-reduction buffer A with 2 mM of reduced glutation (GSH), 0.2 mM of oxide glutation (GSSG) and 1 mM of DTT was used for the renaturation. It was cooled in ice during 30 minutes and added slowly to the purified fusion proteins until the urea was diluted at 1.3 m (dilution factor 6X) and commence the oxidative renaturation. After intense agitation, the fusion proteins were left to rest at 4° C during all night to allow them to reach their thermodynamically stable structures. The samples were kept dialyzing all night in buffer S (250 mM NaCl, 20 mM Tris-HCl 20% saccharose, pH 8.0). A centrifugation was performed at 10000 g during 20 minutes at 4°C, the optical density was measured to calculate the final protein concentration, the preparations were lyophilised and reconstituted in 0.1% of BSA/4 mM HCl before freezing.

### Biological activity of the soluble and collagen-bound proteic forms

The biological activity of the F1 and F2 soluble forms was determined in endothelial cells of human veins (HVEC) by means of a series of proliferation tests. The HVECs were cultivated in Dulbecco Eagles culture medium (DMEM) (GIBCo) supplemented with 1% penicillin/streptomycin and 10% of bovine foetal serum (FBS) (GIBCo), at 37° C and 5% CO₂. Once the culture confluence in mono layer has been reached, the cells were trypsinised and later sown on 24 welled plates (Costar) at a density of 5x10³ cells/well. Once the cells reached 50-60% confluence, the DMEM was substituted by DMEM at 0.5% of FBS and the cells cultivated during 24 hours under these starvation conditions. Serial dilutions of the rhbEGF fusion proteins recently renatured were progressively added to the wells and incubated during 16 hours, followed by the addition of tritium thymidine (³H-thymidine) (1µCi/well, with specific activity of 2 Ci/millimol, 74 GBq/millimol) (ICN) and an additional incubation of 4 hours. The commercial rhbFGF (R&D Systems) was used as standard control. After incubation, the cells were washed three times with saline phosphate buffer (PBS), and smoothed with 150 µl/well of lysis buffer (Solvable, ICN) at room temperature during 10 minutes. The resultant cellular extracts corresponding to DNA were analysed in a scintillation equipment (Beckman 2000).

For the specific case of the rhbFGF-F2, a second biological activity measurement was performed. 500 µl of type I collagen were taken (3.0 mg/ml, pH 7.0) coming from an extraction with pepsin performed in a bovine tendon (see Nimni, M.E., Biorheology (1980), 17: 51-82), leaving to dry until the collagen molecules aggregated in fibres on the base of each well of a culture plate of 24 and were later covered with 0.2% of BSA. The renatured rhbFGF-F2 fractions in PBS were added to the collagen covered wells, incubated at 37°C during 2 hours and later the wells were washed three times with DMEM. The HVEC (1.5×10³ per well in 0.5% FBS/DMEM) were sown and incubated during 20 hours at 37° C before the addition of ³H-thymidin to determine the DNA synthesis as proliferation measurement (as has been previously described).

### Analysis of the specific collagen binding of the rhbFGF-F2

Two different procedures were used to check the rhbFGF-F2 fusion protein affinity to collagen. In the first method, the recombinant fusion protein was first immobilized in a Ni-NTA column to later pass type I collagen (biosynthetically marked with ³H-prolin) through the column, and subsequently eluated with a phosphate buffer lineal concentration gradient (from 0.15 to 2 M) or urea (from 0 to 4 M).

In the second method, the inclusion bodies containing the rhbFGF-F1 and rhbFGF-F2 constructions were isolated by centrifugation and radioactively marked by a method developed in our laboratory (see Cheung D.T. and collaborators, Anal. Biochem. (1981), 116: 69-74). Briefly, the inclusion bodies were dehydrated with DMF (Sigma) in glass test tubes, to which 0.5 ml of ³H-NaBH4 (100 mCi, Amersham)/DMF solution was subsequently added. This suspension is immediately agitated and left to rest during 1 hour at room temperature. The decantants are washed three times with DMF and later PBS. After the purification and renaturation of the fusion proteins as has been previously described, the bioactivity is determined. In culture plates with 24 wells, 200 µl of type I collagen (3.0 mg/ml) were incubated during 30 minutes at 37° C until they formed a fibrillose structure. Next, 20µl of ³H-rhbFGF-F1 and ³H-rhbFGF-F2 were added in serial dilutions on the fibrillose collagen gels and left to rest during 1 hour at 37° C. The gels are washed with 2.0 ml PBS three times, 30 minutes each. 0.5 ml of collagenase A is added (100 U/ml, Boehringer Mannheim) and incubated during 1 hour at 37° C. 20 µl of the resultant solution is taken and measured in the scintillation counter.

### Applicability in wound cicatrization

In order to determine the applicability of rhbFGF-F1 and the possible improvement introduced by the rhbFGF-F2 in the cicatrization of wounds, 24 Wistar male rats are used, with a weight comprised between 300 and 350 grammes. As is known, the diabetic animals signify an excellent experimental model for the study of cicatrization processes of wounds, since said process is delayed and even in some cases, impeded in these animals. Due to this, we used 12 diabetic rats induced in our laboratory by intramuscular injection of 45 mg/kg streptozotocine (Sigma). The result of this dose is a 100% of hyperglucemia and 0% of mortality. The glucose content in blood was daily measured in these animals and it was considered that they had developed diabetes when, during three consecutive days, they presented 350 mg/dl or more of glucose in serum.

Under anaesthesia with sodium pentobarbital, the back of the animals was shaved, the skin sterilized with ethanol at 70%, and eight 5 mm² wounds were inflicted, each one with a depth up to the fleshy panniculus. Each wound was separated by at least 1.5 cm from the other. Two wounds per animal (normal and diabetic) were treated with saline solution (0.05 M sodium acetate, 0.001 M EDTA, 0.001 M CaCl, 0.09 M NaCl, pH 6.0), 100 µl (1 µg/µl) of commercial hbFGF (Chemicon International), or the same rhbFGF-F1 or rhbFGF-F2 dose. The growth factors and their saline diluent were also applied to the wounds mixed in a a type I collagen gel coming from the tail tendon of rats (Collaborative Research). The collagen is dissolved in acetic acid 0.1 M, the pH neutralized at 7.2, and the resultant solution mixed with DMEM to obtain a final concentration of 0.35 mg/ml. The mixture of the bFGFs or of the saline diluent and the type I collagen solution are incubated at 37°C during 15 minutes to obtain a final collagen gel matrix. Daily applications are made under anaesthesia during three days, to later carry out a macroscopic follow-up of the cicatrization. Other animals are sacrificed by means of a dose of 75 mg/kg of Nembutal after 10 or 21 days from infliction of the wounds. Parts of the skin subjected to treatment are taken and histologically processed, dying with hematoxylin-eosin, and immunocitochemically with type IV anti-collagen antibodies, anti-fibronectin and anti-laminin developed in rabbits (Chemicon International), in order to determine the evolution of the cicatrization process.

### Results

### Recombinant construction of the hbFGF-F1 and -F2 fusion proteins

As is shown in Figure 1, we have developed by means of RT-PCR techniques and recombinant DNA technology, two hbFGF fusion proteins. One of these incorporates a molecule domain with high binding affinity to type I collagen. It deals with the incorporation to the protein of a decapeptide coming from the von Willabrand factor (vWF), responsible for the recognition of collagenic sequences in blood vessels, and in which the original cysteine has been replaced by a methionine, in order to aid the renaturation process. In this way, the incorporated decapeptide is the one corresponding to the WREPSFMALS sequence, and the resultant fusion protein, rhbFGF-F2, develops a special capacity of specifically binding to type I collagen.

### Expression, solubilization and purification of the fusion proteins

The rhbFGFs fusion proteins have been expressed at high levels in transformed bacteria (BL21-DE3 stock) that carry the polymerase T7 system. In the presence of 1 mM of IPTG, the rhbFGFs expression levels reached 50% of the total cellular protein, as was determined by electrophoresis in SDS-PAGE gel (Figure 2). The proteins were isolated from the inclusion bodies gathered after a bacterial cellular lysis. In order to prevent the co-sedimentation of the cells and cellular residues, the initial lysis was improved with the combination of an enzymatic treatment with lysozyme (0.4 mg/ml), homogenisation and sonication. Due to the fact that the proteins with origin in inclusion bodies contain variable amounts of disulfite type intercatenary bindings, 10 mM of β-mercaptoethanol was added to the lysis buffer in order to break such bindings.

In spite of the fact that strong denaturants such as guanidine-HCl 6 M and urea 8 M are quite effective in the solubilization of FGFs type proteins, coming from inclusion bodies, the guanidine 6 M was the one that presented the best performance. After the solubilization and before the renaturation, the purification process was approached, with the idea of first eliminating other types of soluble proteins that could come to offer undesirable interactions with the bFGFs, as well as preventing degradations due to the contact with renaturated soluble proteinases present in the same mixture. The purification of rhbFGF-F1 and -F2 was performed by Ni-NTA chromatography carried out under denaturant conditions in the presence of guanidine 6 M. In order to prepare the proteins for the subsequent renaturation steps, the samples bound to the column were washed with urea 8 M, pH 8.0, followed by a more intense wash with urea 8 M, pH 6.5, followed by a final elution of the purified proteins with urea 8 M at pH 4.0. Approximately, the performance was of purified 6 mg of rhbFGF-F1 and 8 mg of rhbFGF-F2, as from 100 ml of bacterial culture.

### Renaturation of the rhbFGF fusion proteins

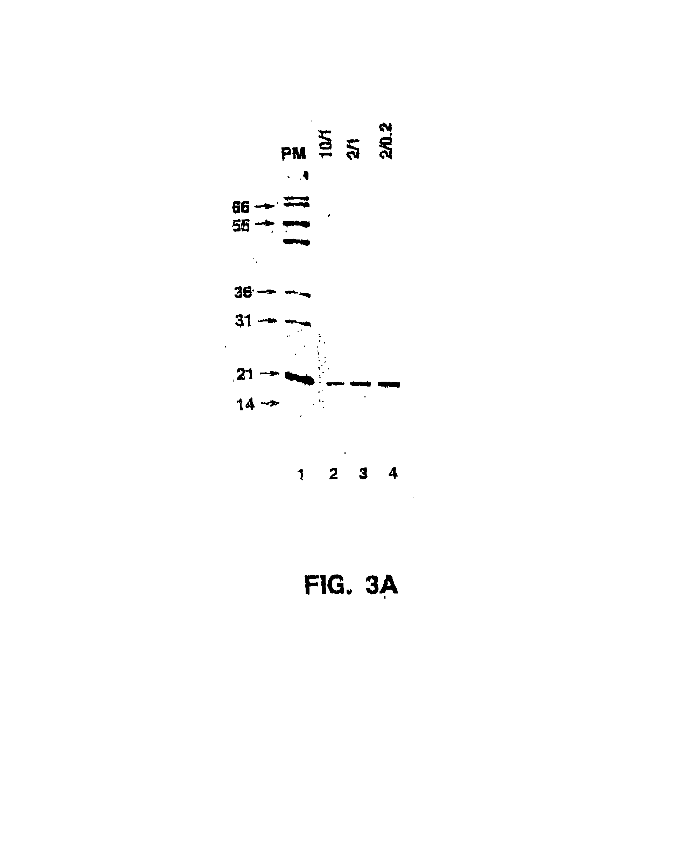

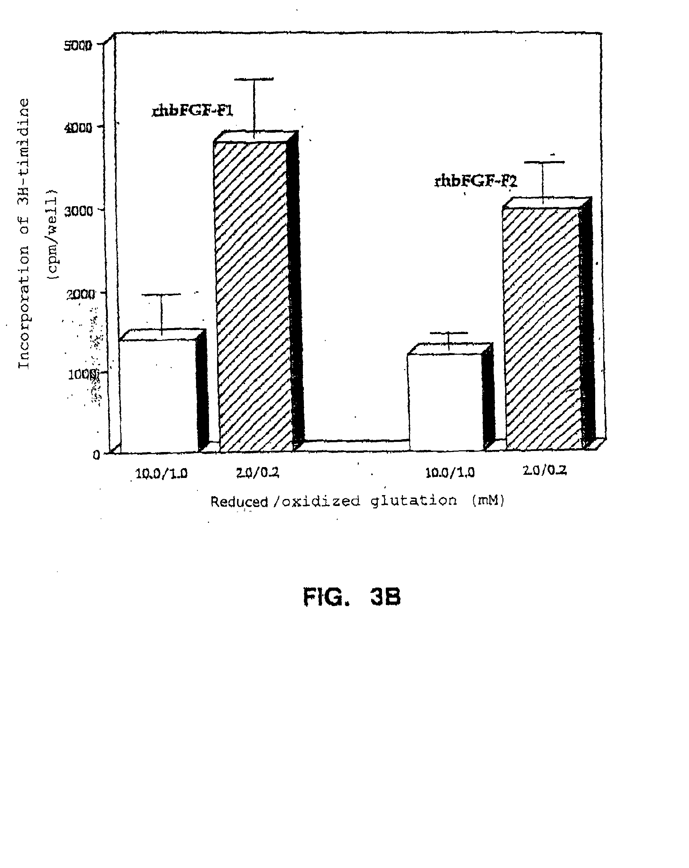

Since the control of the oxide-reduction conditions is a key factor in the correct renaturation of fusion proteins, we have tested various concentrations of GSH and GSSG in order to increase the rate and performance of correctly renatured F1 and F2. As is shown in Figures 3A and 3B respectively, a redox system made up of 2.0 mM/0.2 mM of GSG/GSSG provides the maximum performance of both proteins, as well as the maximum specific relative biological activity when proliferation was measured using HVEC cultures.

### rhbFGF fusion proteins concentration

Associated to the actual kinetics and thermodynamics of the renaturation of proteic monomers, the formation of aggregates (coming from the interaction between proteic precursors half way in the renaturation) compete extraordinarily with the renaturation of bFGF type proteins. Consequently, and since the aggregation implies a decrease in the resultant protein concentration, we established in each renaturation test a concentration limit that was of 20 µg/ml for rhbFGF-F1 and somewhat lower, 18 µG/ml, for rhbFGF-F2, due to the increase of hydrophobic interactions associated to the decapeptidic sequence of vWF.

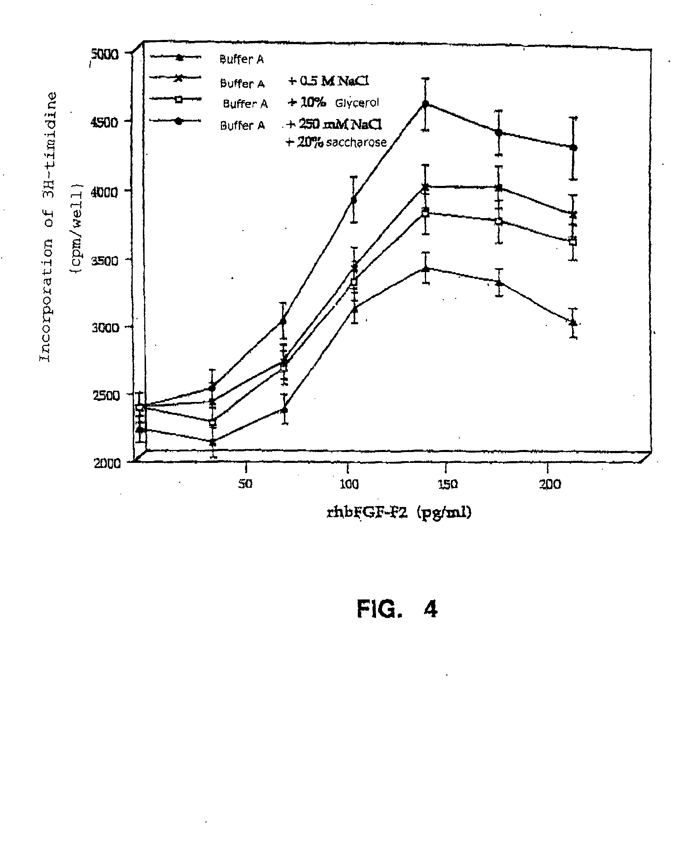

Though the elimination of solubilising agents and the components of the renaturation process are eliminated by simple dialysis, parameters such as the dialysis buffer, the pH, the ionic powers and stabilizing agents of the proteic structure, are of special importance to prevent the precipitation of the recently renatured protein. The greatest performance in terms of active soluble protein after the dialysis was obtained with buffer A, supplemented with 20% saccharose at pH 8.0. At pH 6.5, precipitation was produced and at pH 11.0, though no precipitation whatsoever occurred, the specific biological activity was totally lost. The result as regards specific biological activity values varied according to the different dialysis conditions tested, as is shown in Figure 4 for protein rhbFGF-F2. Organic solvents were in no case added to the dialysis buffer to prevent aggregation, which speaks in favour of the suitability of the tested dialysis mixtures.

After determining the final proteic concentration, proteins rhbFGF-F1 and rhbFGF-F2 were lyophilised. The reconstitution of the proteic lyophilisation for its subsequent use was made with a 4 mM HC1 and 0.1% of BSA solution. Under these conditions however, we detected a loss of biological activity of two units as regards the recently dialyzed samples. The problem is solved when the dialyzed samples are simply frozen at -20° C.

### Biological activity of the fusion proteins

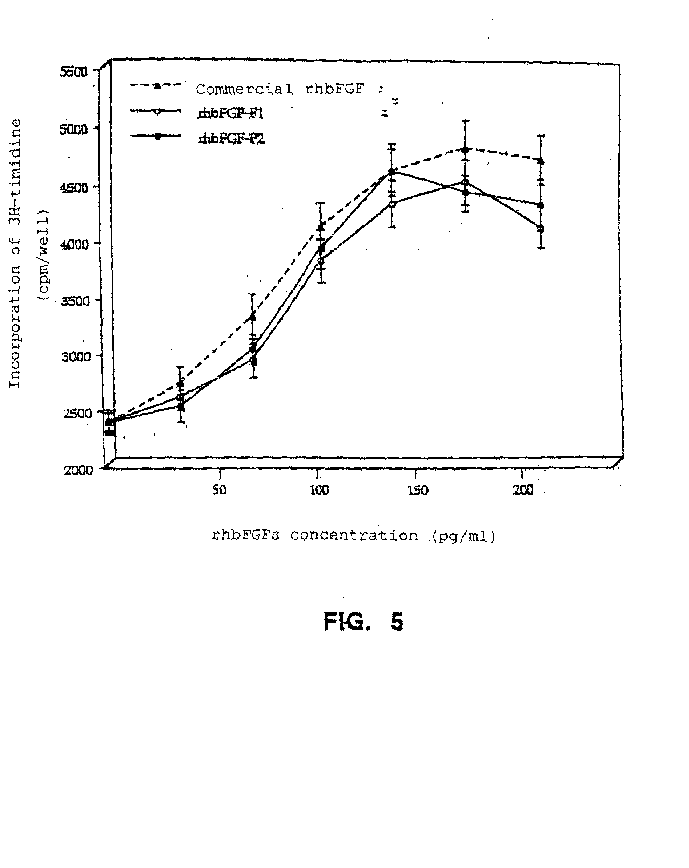

The biological activity of the rhbFGF-F1 and rhbFGF-F2 fusion proteins was checked in an *in vitro* proliferation test using HVEC cellular cultures, where the commercial rhbFGF served as positive control. As is shown in Figure 5, dialyzed proteic fractions were added to cellular cultures lacking serum during 24 hours, from 5 to 30 µl/ml, in concentrations comprised between 50 and 200 pg/ml during 16 hours, after which proliferation activities were obtained that were comparable to those achieved with the commercial growth factor.

### Binding capacity of rhbFGF-F2 to type I collagen

The check of the rhbFGF-F2 fusion protein affinity with type I collagen was approached by means of two strategies. In the first place, 100 ng of the recently dialyzed protein was loaded in a Sephadex G15 column covered with collagen. In principle, all the loaded protein remained bound to the column according to a proteic analysis immediately carried out on the eluant. The intents to eluate the protein of the column using a NaCl gradient or 2 M urea, solutions generally employed in the elution of proteins under these conditions, were useless, suggesting that the rhbFGF-F2 had strongly bound to the column.

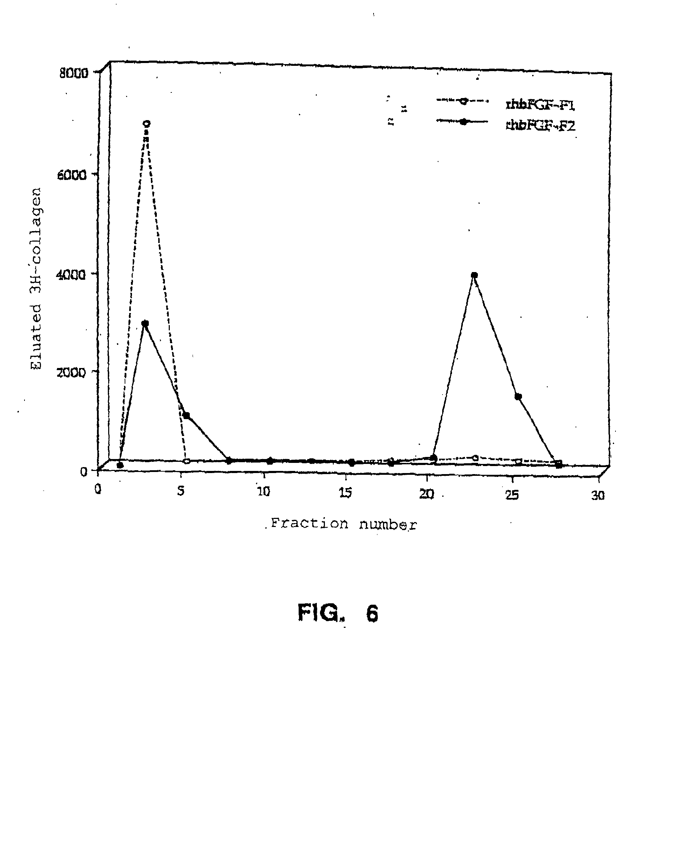

In a second strategy, the rhbFGf-F2 was loaded in a Ni-NTA column, to subsequently make a solution of type I ³H-collagen pass through it. The most part of the loaded radioactive collagen remained retained in the column, according to an immediate proteic analysis carried out on the eluant. The column was washed with a NaCl lineal gradient from 0.15 to 2 M, without achieving any radioactive collagen elution, which demonstrated the strong binding to the rhbFGF-F2. As is shown in Figure 6, only the application of an urea lineal gradient from 0 to 4 M was capable of the eluation of all the ³H-collagen. On the contrary, when the process is repeated with the rhbFGF-F1 (it lacks the vWF amino acidic domain), which, in this case, also served as positive control of the technique, only traces of ³H-collagen were retained by the column. These results demonstrated that the amino acidic domain of the specific binding to type I collagen is responsible for the high specific interaction developed by the rhbFGf-F2 to the collagen.

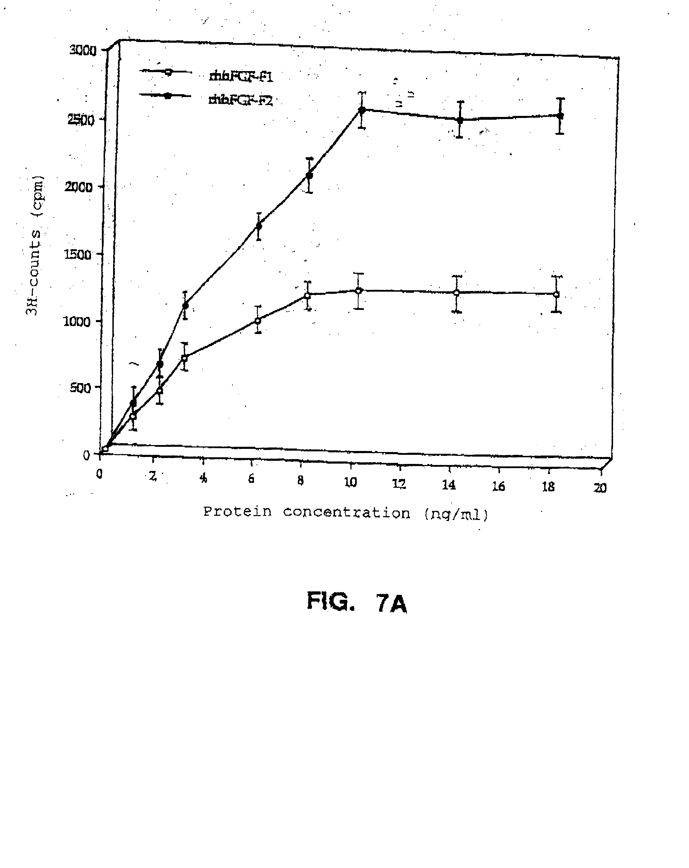

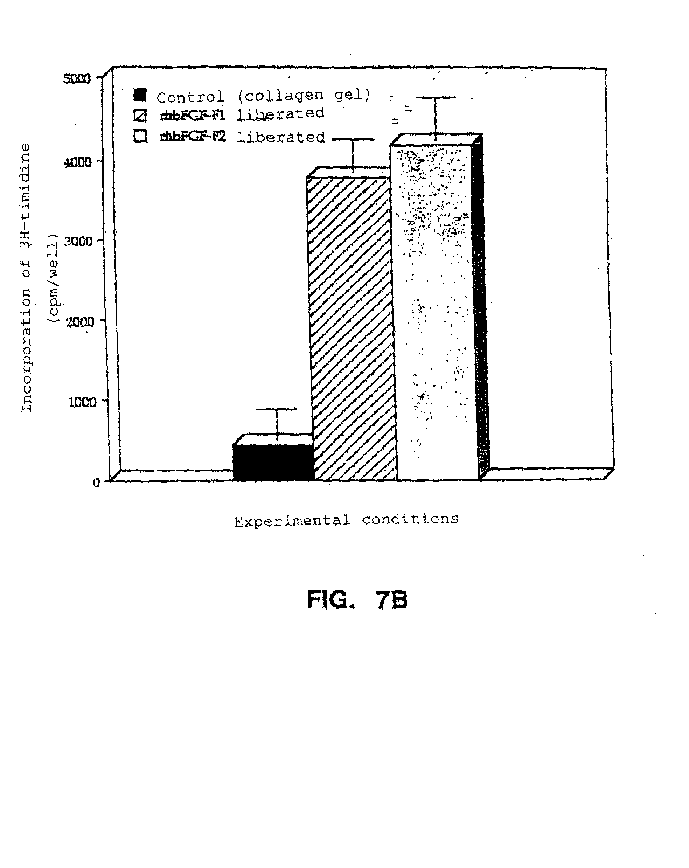

The conclusion of these studies additionally allowed us to demonstrate two main facts: i) that the binding of the rhbFGF-F2 to the type I collagen was a dependant dose, the binding of the rhbFGF-F1 being of very low affinity and motivated by non specific interactions, as is shown in Figure 7A; and ii) that when the rhbFGF-F2 is liberated from the type I collagen by means of enzymatic digestion with collagenase, the fusion protein maintains its biological activity complete, as is shown in Figure 7B.

This set of experiments demonstrates that the molecular domain of the specific binding to type I collagen incorporated to the rhbFGF-F2 protein, causes the fusion protein to be sequested by the collagen matrix, and that the specific collagenic digestion of the same liberates the rhbFGF-F2 with its biological activity intact.

### Effect of the fusion protein application on the cicatrization of wounds

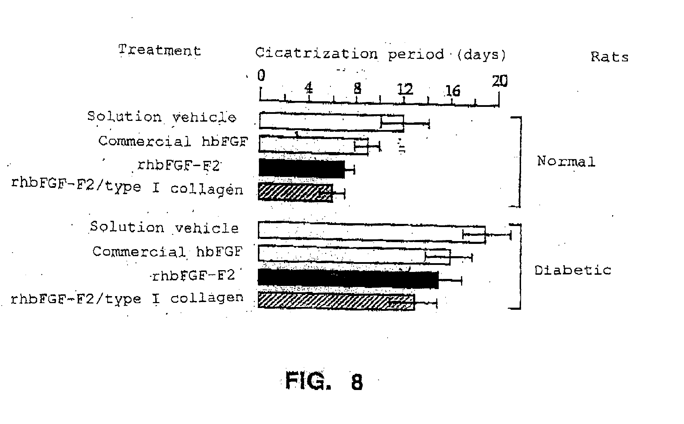

We have tested the two fusion proteins obtained in the rate of the cicatrization of skin wounds on normal and diabetic rats. As is shown in Figure 8, the wounds on normal animals, ectopically treated only with the factor solution vehicle (saline solution), with once-a-day applications during three days, starting on the same day of the injury, cicatrized after 12 days. On diabetic animals, the complete cicatrization was delayed for 7 days with respect to normal rats, what indicated that diabetes negatively affects the cicatrization process (p<0.001). When the wounds were treated with 1 µg of commercial hbFGF, the cicatrization rate was significantly accelerated in 3 days, both on normal and on diabetic animals. When the wounds were treated with 1µg of the fusion protein with binding affinity by collagen I, rhbFGF-F2, the cicatrization process was reduced in 2 days for normal rats and in 1 day for diabetic rats, when compared with the treatment with commercial hbFGF. Very significant was the result obtained when the wounds were created with 1 µg of rhbFGF-F2 mixed with collagen I. Under these conditions, the cicatrization process was reduced even more, specially on diabetic rats (2 days compared with only rhbFGF-F2, and 3 days compared with the commercial hbFGF) . The treatment of wounds with the rhbFGF-F1 obtained similar results to those obtained with the commercial hbFGF, both on normal and on diabetic animals. The type I collagen alone behaved in similar manner as the saline solution in terms of cicatrization.

### Discussion

With the object of directing the hbFGF to specific cellular-tissular spaces and increasing the average life of this powerful mitogenic growth factor, we have designed a fusion protein that incorporates a molecular domain of ten amino acids, which confers the factor with the capacity of binding with very high affinity to type I collagen. This fusion protein, which we have named rhbFGF-F2, may be led directly to the MEC or be mixed with implantable collagenic matrix (in the form of gels or solids). The amino acidic domain used in this construction has been the decapeptide WREPSFMALS, that corresponds to a modification of the von Willebrand factor, which presents a high specific binding affinity to collagen or gel (see Takagi J. and collaborators, Biochem. (1992), 31: 8530-8534). We have demonstrated that the substitution in the decapeptide of the cysteine by the methionine in position 7 (to improve the renaturation conditions) does not interfere in said affinity. With regard to the fusion protein itself, the rhbFGF-F2 does likewise, not present any decrease in its renaturation process, when compared with the rhbFGF-F1 (it lacks the amino acidic domain), maintaining a biological activity that is comparable to the commercial hbFGF, when the proliferation rate is measured in HVEC cultures.

The renaturation of proteins aggregated in bacterial inclusion bodies to biologically 'active conformations, requires a series of steps that are interdependent one with the others: the solubilization, the oxidative renaturation and the elimination of denaturants, each one of which represents a challenge for its optimisation (see Handl C.E. and collaborators, Protein Expr. Purif. (1993), 4: 275-281; and Cardamone M. and collaborators, Biochem (1995), 34: 5773-5794). Our results demonstrate a notable improvement on each one of them. 1) We have determined that a strong ionic denaturant, guanidine 6 M, was more effective than urea 8 M in the solubilization of inclusion bodies. However, in the proteic analysis, after each renaturation step with guanidine-HC1 solution, we detected that the fusion protein activity was notably lower than when urea was employed. Some authors have demonstrated that the guanidine-HC1 might negatively interfere with the renaturation process (see Cardamone M. and collaborators, Biochem (1995), 34: 5773-5794). For this reason, we have initially used in our study guanidine-HCl as denaturant for solubilization of the inclusion bodies and to optimise the proteic performance, to be later substituted by guanidine-HCl with urea 8 M to develop the purification. 2) The oxidative renaturation was carried out in urea 1.3 M, pH 8.0. 3) Since the removal of the denaturants by simple dialysis causes abundant proteic precipitation, we have performed a six-time initial dilution of the denaturant (from urea 8 M to urea 1.3 M) on an oxide-reduction buffer, which is capable of maintaining both the urea concentration and the redox conditions during an interval of 48 hours. The result is that, during this time, the soluble proteins rearm in their native conformations and the biological activity considerably increases without any precipitation. Small molecules, such as glycerol, saccharose, poly-ethylenglycol PEG 200- 2000, bovine serum albumen or L-arginine, are generally added to the dialysis buffers to avoid the proteic precipitation (see Rudolph R. and Lilie H., FASEB J. (1996), 10: 49-56). The concentration of salts and the pH are also key factors to be considered in the dialysis, since the proteic stability depends on the ionisation states of lateral residues (see Schumann J. and collaborators, Protein Sci. (1993), 2: 1612-1620). Consequently, we have determined that the ideal dialysis conditions for the rhbFGFs fusion proteins are 250 mM NaCl, 20% saccharose and pH 8.0. As has been described (see Gary A.M. and collaborators, Science (1990), 247: 1328-1330), the inclusion of auxiliary molecular domains might hinder the renaturation process. However, with the adaptations performed, molecular, oxide-reduction, pH, and protein concentration levels, addition of small molecules and dialysis conditions, we have improved the performance for the obtaining of rhbFGF-F1 and -F2 proteins in 30%.

The bFGF is a multifunctional growth factor which biological properties suggest a main role in the cicatrization of wounds. This is a complex process that starts with an inflammatory phase and the liberation of components from the blood to the wound, that activate a cascade of blood clots formation and provide a matrix for the affluence of inflammatory cells. Subsequently the tissue for the new flesh commences, to form a dense population of fibroblasts and macrophages embedded in a large matrix of collagen, fibronectine and hyaluronic acid, that is to say, MEC. Endothelial cells proliferate in the wound and new blood vessels are formed (angiogenesis)- in order to supply nutrient and oxygen to the injured zone, which is an essential supply for the synthesis, the deposit and the organisation of the MEC (see Satoh H. and collaborators, Jpn. J. Pharmacol (1997), 73: 59-71). It has been demonstrated that the bFGF is a powerful mitogenic for endothelial vascular cells. Additionally, it is produced and liberated in damaged tissues, contributing to the proliferation of fibroblasts as well as to the induction to the MEC synthesis by said cells (see Clark R.A.F, Molecular and Cellular Biology of Wound Repair (1996), 3-50).

Since the bFGF is a pleiotropic agent that stimulates, inhibits and modulates cellular processes in a time- and dose-dependent way, it is essential to control its liberation to the desired location and its bioavailability whilst its action as therapeutic agent is necessary. In our study, the treatment of wounds with rhbFGF-F2 accelerated the cicatrization process both in normal and in diabetic rats, obtaining the maximum significance when the fusion protein was applied to the wound combined with type I collagen in the form of gel. This dressing did not only achieve the acceleration of the closing of the wound in normal rats in comparison with commercial hbFGF, but also cancelled the delay produced in the cicatrization response in diabetic animals (complete cicatrization after 13 days, approximately the same time the wounds of normal rats need for cicatrizing when treated only with the saline solution). According to the results confirmed *in vitro,* we gather that the specific collagen binding molecular domain would be incorporating in the rhbFGF-F2 protein the capacity of specifically binding to type I collagen, which is serving as carrier. Once this dressing (collagen + factor) is placed on the fresh wound, the presence of collagenase type enzymes, the presence of which during the first days of cicatrization has been demonstrated (see Edelman E.R. and collaborators, Biomaterials (1991), 12: 619-629), would be causing a slow liberation of the rhbFGF-F2 from the collagenic matrix to which it is bound towards the large cellular component implicated in the cicatrization (fibroblasts, in charge of synthesizing more collagen matrix, endothelial cells, inflammatory cells), thus obtaining a greater and improved availability of the factor by the target cells. In this case, the type I collagen would be acting as physiological modulator in the liberation of the protein towards the target elements. This fact has been recently discussed by the authors of this patent for a growth factor rhTGF-β1 (see Gordon E.M. and collaborators, Hum. Gene Ther. (1997), 8: 1385-1394; and Andrades J.A. and collaborators, Exp. Cell Res. (1999), 250: 485-498).

Due to the fact that the mitogenic activity of the bFGF is rapidly lost when the factor is injected, it has been demonstrated that when the protein is combined with specific materials (for example, heparin) an increase of the proteic stability is achieved, as well as the preservation of its biological activity (see Edelman E.R. and collaborators, Biomaterials (1991), 12: 619-629). The fact that the maximum cicatrization rate, both with normal and with diabetic animals is obtained when the rhbFGF-F2 was applied combined with type I collagen, reinforces the idea that the amino acidic domain WREPSFMALS is incorporating a greater and/or longer lasting biological activity to the resultant fusion protein.

## Claims

1. Basic fibroblastic growth factor with a specific collagen binding domain, obtained by recombinant engineering (rhbFGF-F2) and **characterised in that** it includes a ten amino acids domain corresponding to the modified von Willebrand factor that permits its combination with type I collagen in gel or solid phase.

2. Basic fibroblastic growth factor with a specific collagen binding domain (rhbFGF-F2), according to Claim 1, that in combination with type I collagen, serves to transport the protein towards the required repair locations, such as ulcers, burns of different ethyology and bone fracture repairs.

3. Basic fibroblastic growth factor with a specific collagen binding domain (rhbFGF-F2), according to Claim 1 that, by itself or combined with type I collagen, is applicable for accelerating the cicatrization of wounds on soft tissues, such as skin and dermal wounds, both with total or partial incision.

4. Use of the basic fibroblastic growth factor with a specific collagen binding domain (rhbFGF-F2), according to Claim 1, in ectopic application compositions for the cicatrization of wounds.

5. Preparation method by recombinant engineering of the basic fibroblastic growth factor with a specific collagen binding domain (rhbFGF-F2) according to Claim 1, **characterised by** the incorporation to an auto-response vector of a DNA purified fragment that includes:
- A DNA sequence that codes for a purification signal of the type (His)m, ribonuclease S, or hemaglutinine A.
- A DNA sequence that codes for a proteinase of the thrombin, plasmin, tripsin, elastase, pepsin, chimosin or thermolisin group.
- A DNA sequence that codes for an extracellular matrix binding region of the collagen, fibronectin or cellular surface type.
- A DNA sequence that codes for the active fragment of the human basic fibroblastic growth factor.

6. Method to obtain the basic fibroblastic growth factor with a specific collagen binding domain (rhbFGF-F2), mature and active, coded by a DNA fragment according to Claim 4, that comprises the following phases:
- the isolation of the proteic fraction that contains the mature rhbFGf-F2 region;
- the protein expression in transformed bacteria that carry the polymerase T7 system, in the presence of IPTG;
- the denaturation of the proteic fraction in a denaturant buffer with urea, Tris-HCl, NaCl and NP-40, at pH 8.0;
- the purification of the fusion protein by Ni-NTA chromatography by means of urea at different pH's;
- the dilution of the mature rhbFGF-F2 polypeptide at an optimum concentration of 0.2 mg/ml;
- and the renaturation by dialysis in an oxide-reduction and DTT buffer, followed by a buffer with Tris-HC1, NaC1 and saccharose, at pH 8.0
